# EUROPÄISCHE PATENTSCHRIFT

(11) **EP 3 763 342 B1**
(45) Veröffentlichungstag und Bekanntmachung des Hinweises auf die Patenterteilung: **22.07.2026**
(21) Anmeldenummer: 20194913.8
(22) Anmeldetag: 04.02.2014
(51) Int. Cl.: A61F 9/008

(54) **ERZEUGUNG GEKRÜMMTER SCHNITTE IM INNEREN DER AUGENHORNHAUT**
CREATION OF CURVED CUTS IN THE INSIDE OF THE EYE CORNEA
RÉALISATION D'ENTAILLES COURBÉES À L'INTÉRIEUR DE LA CORNÉE

(30) Priorität: 14.03.2013 DE 102013204496
(43) Veröffentlichungstag der Anmeldung: 13.01.2021
(62) Teilanmeldung aus: 14703324.5
(73) Patentinhaber: Carl Zeiss Meditec AG, 07745 Jena (DE)
(72) Erfinder: BISCHOFF, Mark, 07749 Jena (DE); STOBRAWA, Gregor, 07743 Jena (DE)
(74) Vertreter: Patentanwälte Geyer, Fehners & Partner mbB

(56) Entgegenhaltungen:
- DE-A1- 10 202 036
- DE-A1- 102008 056 488
- DE-A1- 102008 062 658
- US-A1- 2004 243 112
- US-A1- 2012 078 240
- KARSTEN KOENIG ET AL: "Intratissue surgery with 80 MHz nanojoule femtosecond laser pulses in the near infrared", OPTICS EXPRESS, vol. 10, no. 3, 11 February 2002 (2002-02-11), pages 171 - 176, XP055108893, ISSN: 1094-4087, DOI: 10.1364/OE.10.000171

## Beschreibung

Die Erfindung bezieht sich auf eine Vorrichtung zum Isolieren eines Lentikels in der Hornhaut eines Auges, die aufweist eine Laserstrahlquelle, die ausgebildet ist, gepulste Laserstrahlung abzugeben mit einer in die Hornhaut eindringenden Wellenlänge, einer Strahlformungseinrichtung, die aufweist eine Strahloptik, welche die gepulste Laserstrahlung in die Hornhaut in einen Fokus bündelt, und einer Strahlablenkeinrichtung, die einen Fokus der Strahlung in der Hornhaut verschiebt, wobei eine Steuereinrichtung vorgesehen ist, die ausgebildet ist, die Laserstrahlquelle und die Strahlformungseinrichtung anzusteuern, um in der Hornhaut ein Lentikel zu isolieren, welches durch eine Schnittfläche begrenzt ist.

Die Erfindung bezieht sich weiter auf ein Verfahren zum Isolieren eines Lentikels in der Hornhaut eines Auges, wobei in der Hornhaut mindestens eine Schnittfläche festgelegt wird, die das Lentikel umgrenzt und die Schnittfläche in der Hornhaut durch die Abgabe gepulster Laserstrahlung erzeugt wird, wobei gepulste Laserstrahlung verwendet wird, die eine in die Hornhaut eindringende Wellenlänge hat und ein Fokus der Laserstrahlung in der Hornhaut verschoben wird. Die Erfindung umfasst dabei keine chirurgische Behandlung des lebenden menschlichen oder tierischen Auges.

Für die Abbildungseigenschaften des Auges ist die Form der Vorderfläche der Augenhornhaut von Bedeutung. Es ist deshalb seit langem bekannt, für eine Fehlsichtigkeitskorrektur die Augenhornhaut zu verändern, mit dem Ziel die Vorderfläche der Augenhornhaut und damit deren Brechungseigenschaften zu modifizieren und so eine Fehlsichtigkeit auszugleichen. Im Stand der Technik wurden dazu Operationsverfahren entwickelt, welche an der Augenhornhaut eine Lamelle lösen, diese abklappen und dann aus dem derart freigelegten Inneren der Augenhornhaut Material abtragen. Anschließend wird die Lamelle wieder zurückgeklappt und die Augenhornhaut hat aufgrund des Materialabtrages eine anders geformte Vorderfläche. Dieses Korrekturprinzip ist im Stand der Technik u. a. mit der Bezeichnung LASIK abgekürzt und sei nachfolgend als lamellenlösende Fehlsichtigkeitskorrektur bezeichnet. Das Lösen der Lamelle erfolgte in frühen Ausführungsformen mittels eines mechanischen Keratoms. Die Augenhornhaut wird durch ein ebenes Kontaktglas flachgedrückt und mittels des mechanischen Keratoms ein die Lamelle erzeugender Schnitt ausgeführt. In einer Weiterbildung werden mittlerweile sogenannte Laserkeratome verwendet. Hierzu ist u. a. ein Laserkeratom der Ziemer Ophthalmic Systems AG, Port, Schweiz, bekannt. Es ist hinsichtlich seiner Strahlablenkung zur Erzeugung der abzuklappenden Lamelle ausgebildet. Ein weiteres Laserkeratom wurde von der Intralase Inc., USA, entwickelt, die mittlerweile der Abbott Laboratories, Illinois, USA, gehört. Beide Laserkeratome arbeiten mit gepulster Laserstrahlung, wobei sich Repetitionsraten und Pulsenergien unterscheiden.

Die chirurgische Fehlsichtigkeitskorrektur wurde weiterentwickelt zu Verfahren, die in der Hornhaut Material isolieren und extrahieren. Das Material hat üblicherweise die Form eines Lentikels, weshalb diese Verfahren hier lentikelextrahierende Verfahren bzw. Vorrichtungen genannt werden. Auch wird das zu isolierende und extrahierende Volumen der Anschaulichkeit halber mit der Bezeichnung "Lentikel" versehen, auch wenn in bestimmten Anwendungsfällen ein nicht lentikelförmiges Volumen zu isolieren und extrahieren ist.

Die eingangs genannten Vorrichtungen und Verfahren beziehen sich auf das Prinzip der Lentikelextraktion. Die lentikelextrahierenden Vorrichtungen und Verfahren haben den Vorteil, dass die Vorderfläche der Hornhaut in einem sehr viel kleineren Bereich verletzt wird. Man benötigt nicht mehr einen nahezu vollständig ringförmigen Einschnitt an der Hornhautvorderfläche, wie er zum Lösen einer das Hornhautinnere freilegenden Lamelle erforderlich ist. Es genügt vielmehr ein kleiner Einschnitt am Rand, der zum isolierten Volumen führt und durch den das isolierte Volumen entnommen werden kann, gegebenenfalls nach vorheriger Zerkleinerung des isolierten Materials. Das Prinzip der Lentikelextraktion erfordert es jedoch, die das Lentikel isolierenden Schnittflächen im Inneren der Hornhaut hochpräzise zu erzeugen. Um eine Fehlsichtigkeitskorrektur zu bewirken, sollte für einen möglichst geringen Gewebeverbrauch weiter mindestens eine der das Lentikel begrenzenden Schnittflächen in einem nicht konstanten Abstand zur Vorderfläche der Hornhaut liegen. Auch hier besteht ein Unterschied zum Ansatz, der eine Hornhautlamelle löst und abklappt, da dort die einzige und lamellenerzeugende Schnittfläche problemlos in konstantem Abstand zur Vorderfläche der Hornhaut liegen kann, also parallel zur Vorderfläche der Hornhaut. Wenn beim lamellenerzeugenden Schnitt die Vorderfläche der Hornhaut mit einem planen Kontaktglas flachgedrückt wird, muss man lediglich eine Schnittfläche erzeugen, die bis auf Randabschnitte parallel zur Fläche des Kontaktglases liegt und ihrerseits auch eben ist.

Die lentikelextrahierende Fehlsichtigkeitskorrektur ist grundlegend in der WO 2004 / 105 660 A1 und der WO 2004 / 105 661 A1 beschrieben. Im Stand der Technik sind darüber hinaus Weiterbildungen bekannt. So offenbart die WO 2005 / 011 547 A1 die Verwendung von Höhenlinien zur schnellen Lentikelisolation, die WO 2008 / 055 697 A1 gibt Berechnungsvorschriften, wie die Grenzflächen des Lentikels, d. h. die erzeugenden Schnittflächen gewählt werden können. Aus dieser Schrift ist es insbesondere bekannt, die das Lentikel begrenzenden Schnittflächen in eine anteriore Flap-Fläche, welche in konstantem Abstand zur Vorderfläche der Augenhornhaut liegt, und in eine posteriore Lentikelfläche aufzuteilen, welche in keinem konstanten Abstand zur Vorderfläche der Hornhaut liegt. Der Abstand der Flächen zueinander und damit ihre Form beeinflusst die Krümmung der Hornhaut nach der Korrektur.

Eine Vorrichtung bzw. ein Verfahren gemäß dem Oberbegriff der unabhängigen Ansprüche ist aus der DE 102 02 036 A1 bekannt.

Die WO 2008 / 055 705 A1 und WO 2008 / 055 706 A1 befassen sich mit der Problematik der Bildfeldkrümmung bei der Verwendung eines nicht planaren Kontaktglases und der Erzeugung von Steuerdaten für das chirurgische Verfahren.

Die Schnittfläche wird üblicherweise durch gepulste Laserstrahlung erzeugt. Dabei werden die Zielpunkte der Laserstrahlung entlang einer Bahnkurve angeordnet, die in der Schnittfläche liegt und letztlich die Schnittfläche vorgibt. Die WO 2008 / 055 698 A1 schildert die Anordnung der Zielpunkte entlang der Bahnkurve, wobei es vorgesehen ist, nicht für jeden in die Augenhornhaut abgegebenen Laserstrahlungspuls auch einen Zielpunkt vorzugeben.

Die WO 2008 / 131 878 A1 widmet sich der Frage, wie nach einem Abbruch eines laserchirurgischen Eingriffs, eine weitere Behandlung erfolgen kann, welche die bereits erzeugten Gewebsveränderungen in der Hornhaut berücksichtigt. Auch die DE102008056488 A1 betrifft ein ophthalmologisches Lasersystem, insbesondere zum Zwecke einer chirurgischen Nachbehandlung der Cornea.

Die WO 2009 / 059 711 A1 und WO 2009 / 059 730 A1 befassen sich mit verschiedenen Profilen des zu entnehmenden Lentikels für bestimmte Fehlsichtigkeitskorrekturen, nämlich eine Hyperopiekorrektur, und geben Mindestwerte für das Lentikel vor.

Die WO 2003 / 059 563 A2 offenbart Betriebsparameter für eine Laservorrichtung zur operativen Fehlsichtigkeitskorrektur mittels Lentikelextraktion. Gleiches gilt für die EP 1 628 606 B1.

Die US 2012 / 0 078 240 A1 beschreibt eine Laservorrichtung und eine Methode zur Durchführung eines refraktiven Eingriffs an einem Auge.

Die DE 10 2008 062 658 A1 betrifft die lasergestützte Keratoplastik, bei der an der Rückseite der Kornea eine Lamelle geschnitten werden soll.

Für das lentikelextrahierende Verfahren ist im Stand der Technik das Femtosekunden-Laserkeratom VisuMax der Carl Zeiss Meditec AG bekannt. Es verwendet einen Femtosekunden-Faserlaser, welcher im infraroten Spektralbereich emittiert und mit einer Pulswiederholrate von 500 kHz Laserpulse abgibt, die in die Augenhornhaut fokussiert werden.

Sowohl für die Qualität einer Fehlsichtigkeitskorrektur als auch für die Akzeptanz bei Patienten ist die Zeit, welche die Schnittflächenerzeugung dauert, von hoher Bedeutung. Mit der Dauer des Eingriffs steigt das Risiko von störenden Augenbewegungen, welche die Genauigkeit der Schnittflächenerzeugung herabsetzen oder sogar dazu führen können, dass gar keine zusammenhängende Schnittfläche mehr erzeugt werden kann und das Verfahren abgebrochen werden muss. Auch ist ein länger dauernder Eingriff eine unerwünschte Belastung für den Patienten.

Bei der lamellenlösenden Fehlsichtigkeitskorrektur wird die Güte der optischen Korrektur durch die Genauigkeit des Volumenabtrages nach dem Lösen der Lamelle wesentlich bestimmt. Die Lage der lamellenlösenden Schnittfläche selbst ist von untergeordneter oder sogar verschwindender Bedeutung. Für die Güte des Resultates einer lentikelextrahierenden Sehkorrektur ist hingegen die exakte Positionierung der Schnittflächen in der Augenhornhaut von hoher Bedeutung. Da die Schnittfläche durch die Verstellung der Fokuslage (Fokus) der gepulsten Laserstrahlung entlang einer Bahn erzeugt wird, ist letztlich die Positioniergenauigkeit des Fokus in der Augenhornhaut wichtig. Hier darf nicht vergessen werden, dass es sich um lebendes Gewebe handelt, das sich mitunter während des Eingriffs verändern kann und auch nicht zwingend linear auf Parameterveränderungen reagiert.

Der Erfindung liegt deshalb die Aufgabe zugrunde, eine Vorrichtung der eingangs genannten Art bzw. ein Verfahren der eingangs genannten Art so weiterzubilden, dass damit eine schnelle Schnittflächenerzeugung bei gleichzeitig hoher Präzision erreicht wird.

Die Erfindung ist in den unabhängigen Ansprüchen definiert. Das beanspruchte Verfahren umfasst keine chirurgische Behandlung des lebenden menschlichen oder tierischen Auges.

Eine Vorrichtung zum Isolieren eines Lentikels in der Hornhaut eines Auges weist insbesondere auf:
- eine Laserstrahlquelle, die ausgebildet ist, gepulste Laserstrahlung abzugeben mit
   - einer Pulsfrequenz von 1,2 MHz bis 10 MHz,
   - einer Pulsenergie von 1 nJ bis 200 nJ und
   - einer in die Hornhaut eindringenden Wellenlänge,
- einer Strahlformungseinrichtung, die aufweist
   - eine Strahloptik, welche ein Bildfeld hat und die gepulste Laserstrahlung in die Hornhaut in einen Fokus bündelt, der innerhalb des Bildfeldes liegt und einen maximalen Durchmesser unter 3 µm hat, und
   - eine Strahlablenkeinrichtung, die den Fokus in der Hornhaut und innerhalb des Bildfeldes verschiebt, wobei der Fokus bei ruhendem Bildfeld entlang einer Bahn wandert,
- eine Steuereinrichtung, die ausgebildet ist, die Laserstrahlquelle und die Strahlformungseinrichtung anzusteuern, um in der Hornhaut durch die Vorgabe der Bahn das Lentikel zu isolieren, welches durch mindestens eine Schnittfläche begrenzt ist, die gegenüber einer Vorderfläche der Hornhaut gekrümmt ist.

In einem Verfahren zum Isolieren eines Lentikels in der Hornhaut eines Auges wird insbesondere
- in der Hornhaut mindestens eine Schnittfläche festgelegt, die das Lentikel umgrenzt und gegenüber einer Vorderfläche der Hornhaut gekrümmt ist,
- eine Bahn festgelegt, die in der Schnittfläche liegt,
- in die Hornhaut gepulste Laserstrahlung abgegeben mit
   - einer Pulsfrequenz von 1,2 MHz bis 10 MHz,
   - einer Pulsenergie von 1 nJ bis 200 nJ und
   - einer in die Hornhaut eindringenden Wellenlänge,
- eine Strahloptik verwendet, welche ein Bildfeld hat und die gepulste Laserstrahlung in die Hornhaut in einen Fokus bündelt, der innerhalb des Bildfeldes liegt und einen maximalen Durchmesser unter 3 µm hat, und
- der Fokus in der Hornhaut und innerhalb des Bildfeldes verschoben, wobei der Fokus bei ruhendem Bildfeld entlang der Bahn wandert.

Die Erfindung kombiniert verschiedene Merkmale, die zusammen die Erzeugung einer Schnittfläche ermöglichen, welche gegenüber der Vorderfläche der Hornhaut gekrümmt ist. Es handelt sich also nicht um eine herkömmliche, lamellenlösende Schnittfläche, sondern um eine Schnittfläche, die in einer Schnittebene durch das Auge, welche die Sehachse oder die optische Achse des Auges enthält, nicht in konstantem Abstand zur Vorderfläche der Hornhaut verläuft. Die Krümmung, genauer gesagt handelt es sich um eine zweidimensionale Krümmung, gegenüber der Vorderfläche der Augenhornhaut ist bezüglich des erfindungsgemäßen Ansatzes auf einen zentralen Bereich um die optische Achse oder die Sehachse des Auges herum bezogen, der beispielsweise einen Durchmesser von nicht größer als 10 mm hat. Dies ist der für die optische Korrektur wesentliche Bereich, und in diesem Bereich haben übliche initiale Laserschnitte lamellenlösender Korrekturprinzipien einen konstanten Abstand zwischen Vorderfläche der Augenhornhaut und der Schnittfläche, welche die Lamelle zum Freilegen des Inneren der Augenhornhaut bewirkt.

Die erfindungsgemäße Kombination geht aus folgenden Gründen über eine einfache Aggregation von Merkmalen hinaus und erreicht eine Kombinationswirkung:
Die einzelnen Laserpulse, welche entlang der Bahn die Schnittfläche bilden, wirken in der Augenhornhaut unterschiedlich, je nach Pulsenergie, Pulsfrequenz und Fokusdurchmesser.

Hinsichtlich der Gewebetrennung durch die gepulste Laserstrahlung können zwei unterschiedliche Arbeitsregime identifiziert werden. Diese unterschiedlichen Prozesse werden nachfolgend mit dem Begriff "Gewebespaltung" und "Gewebeschneiden" bezeichnet.

Fokussiert man Laserpulse unter bestimmten Bedingungen in die Hornhaut, kommt es im Fokusvolumen zu einer Gewebeauflösung, welche Gase freisetzt, die unter hohem Druck stehen und somit mechanische Kräfte auf die Gewebeumgebung ausüben. Das Hornhautgewebe besteht aus einer lamellenartigen Kollagenstruktur, sodass die mechanischen Kräfte nach Erkenntnis der Erfinder Mikrorupturen erzeugen, die entlang der Lamellen verlaufen. Somit entsteht zusätzlich zum primären Prozess der Gewebeauflösung im Fokusvolumen ein sekundärer Effekt, der zu einer Gewebespaltung entlang der Kollagenstruktur führt. Arbeitet man hingegen mit vergleichsweise geringeren Pulsenergien und höheren Repetitionsraten kommt es nicht mehr zum sekundären Effekt der Gewebespaltung, sondern lediglich die im Volumen des Fokus erzeugte Gewebeauflösung bewirkt das Trennen. Das Augenhornhautgewebe wird also weitgehend unabhängig von der Kollagenstruktur geschnitten.

Die Erfinder erkannten, dass zwischen Gewebespaltung und Gewebeschneiden kein abrupter Übergang erfolgt. Es gibt im Parameterraum der Laserpulsapplikation einen Übergangsbereich, in dem mal der eine oder der andere Mechanismus einen mehr oder weniger großen Anteil am Vorgang der Gewebetrennung hat. Weiter zeigte sich, dass in lentikelextrahierenden Verfahren und Vorrichtungen in der klinischen Praxis bislang ausschließlich Parametersätze angewendet wurden, die dazu führten, dass der sekundäre Effekt der Gewebespaltung wesentlichen Anteil am Trennprozess hat. Eine Verwendung ausschließlich gewebeschneidender Parametersätze erfolgt derzeit in der klinischen Praxis ausschließlich im Laserkeratom der Ziemer Ophthalmic Systems AG, Port, Schweiz. Diese Vorrichtung ist jedoch zur Erzeugung von Schnittflächen, welche gegenüber der Vorderfläche der Hornhaut gekrümmt sind, nach öffentlichem Kenntnisstand nicht in der Lage, und das Arbeitsregime bietet, wie bereits eingangs ausgeführt, beim lamellenlösenden Verfahren keinen wesentlichen applikativen Vorteil und ist daher nicht von Bedeutung.

Es muss betont werden, dass die Identifikation der unterschiedlichen Arbeitsregime, d. h. der Parametereinfluss auf die Frage, ob Gewebespaltung oder Gewebeschneiden bei der Lentikelerzeugung überwiegt, erstmals von den Erfindern genannt wurde. Im Stand der Technik findet sich die Unterscheidung der Arbeitsregime bei diesem Verfahren nicht. Auch die Zuordnung der einzelnen Realisierungen des Standes der Technik zu diesen Arbeitsregimen kennt die Literatur nicht.

Die Erfinder erkannten durch Ihre Arbeiten weiter, dass hinsichtlich der zu erreichenden Genauigkeit ein wesentlicher Unterschied zwischen Gewebespaltung einerseits und Gewebeschneiden andererseits genau nur dann besteht, wenn gekrümmte Schnitte durchgeführt werden. Denn die Gewebespaltung verläuft in der Regel immer entlang der Lamellen der Kollagenstruktur des Hornhautgewebes. Bei lamellären Schnitten besteht daher im Bearbeitungsergebnis kein Vor- oder Nachteil zwischen einer lamellären Spaltung und einem lamellären Schnitt. Anders bei gekrümmten Schnittflächen: Denn aufgrund der physiologisch gegebenen Lamellendicke und -struktur ist es nicht möglich, eine gekrümmte Schnittfläche mit hoher Formtreue an eine beliebige Stelle in der Augenhornhaut zu platzieren, wenn mit gewebespaltender Trennung gearbeitet wird.

Mit dem Mechanismus des Gewebeschneidens kann eine sehr viel genauere Schnittflächenpositionierung und insbesondere eine höhere Formtreue erreicht werden. Die Erfinder führen dies darauf zurück, dass bei einem gewebeschneidenden Prozess anders als bei der Gewebespaltung die Schnittfläche nicht zwingend in Grenzflächen zwischen Lamellen der Kollagenstruktur des Hornhautgewebes verläuft, sondern auch innerhalb einer Lamelle angeordnet sein kann. Ein bei der Gewebespaltung in der Lamellenebene erfolgender Vorriss (Voreilen der Spaltung vor der fortschreitenden Laserpulsfolge) wird vermieden und die Trennung erfolgt genau in der Fokusposition. Die Gewebespaltung hingegen kann quasi als Digitalisierung der Schnittflächenposition verstanden werden, wobei die kleinste Einheit die Lamellendicke ist, da eine gewebespaltend erzeugte Schnittfläche dazu tendiert, immer in Grenzflächen zwischen einzelnen Lamellen des Hornhautgewebes zu liegen. Dies ist für Schnitte, die in konstantem Abstand zur Hornhautvorderseite erfolgen, kein Mangel, denn die Lamellenstruktur folgt der Hornhautvorderseite mit guter Genauigkeit. Für gekrümmte Lentikelschnittflächen, deren Abstand zur Hornhautvorderseite je nach Radius und ggf. Winkel variiert, erweist sich diese Art der Schnittflächenerzeugung aber als Nachteil.

Diese Zusammenhänge führen dazu, dass der erfindungsgemäße Parameterbereich hinsichtlich Pulsfrequenz, Pulsenergie und Fokusdurchmesser besonders zuverlässig zu einer Gewebetrennung führt, bei der der Vorgang des Gewebeschneidens weit überwiegt. Die Kombination mit der Erzeugung einer Schnittfläche, welche gegenüber der Vorderfläche der Hornhaut gekrümmt ist und damit zwangsläufig nicht der Grenzfläche zwischen Lamellen der Kollagenstruktur des Hornhautgewebes (welche im Übrigen weitgehend parallel zur Hornhautvorderfläche verläuft) folgt, führt deshalb zu einer besonders genauen Schnittflächenpositionierung und hohen Formtreue. Im erfindungsgemäßen Arbeitsbereich ist der Beitrag der Gewebespaltung zugunsten der Wirkung des Gewebeschneidens reduziert, sodass für die Lentikelextraktion die Schnittfläche und damit die Isolation des Lentikels präziser den vorgegebenen Werten folgen.

Der gewebeschneidende Effekt ist besonders groß, wenn die Pulsenergie weniger als 100 nJ beträgt. Die Pulsenergie liegt im Pulsenergiebereich ist von 10 nJ bis 80 nJ. Für die Erzeugung der gekrümmten Schnittfläche ist es weiter vorteilhaft, dass alternativ oder zusätzlich die Strahloptik ein Objektiv mit numerischer Apertur von mindestens 0,33 in der Hornhaut aufweist. Als besonders günstig hat sich für die Pulslänge der gepulsten Laserstrahlung bei infraroten Wellenlängen ein Wert von nicht mehr als 1 ps herausgestellt.

Die Wellenlänge der verwendeten Laserstrahlung ist so, dass die Laserstrahlung in die Augenhornhaut eindringen kann (Transmissionsgrad ≥ 0,8) und dort mittels nichtlinearer Effekte im Inneren der Hornhaut zur Gewebetrennung führt. Hierfür hat sich eine Wellenlänge von 1030-1060 nm herausgestellt. Alternativ kann auch Laserstrahlung im ultravioletten Spektralbereich zwischen 300 nm und 400 nm eingesetzt werden. Sie hat zwar tendenziell einen höheren linearen Wechselwirkungsanteil (Absorption) als die genannte infrarote Strahlung, ist aber ebenfalls geeignet, wenn der Fokusdurchmesser 2 µm nicht überschreitet und die Pulsfrequenz nicht über 2 MHz liegt. Wenn Laserpulse aus diesem ultravioletten Wellenlängenbereich verwendet werden, können auch Pulslängen von einigen ns wirksam eingesetzt werden.

Die Isolierung des Lentikels erfolgt dadurch, dass der Fokus innerhalb des Bildfeldes der verwendeten Strahloptik verschoben wird und das Bildfeld gegenüber der zu bearbeitenden Hornhaut ruht. Hierin besteht ein Unterschied zum Laserkeratom der Ziemer Ophthalmic Systems AG, bei der ein Mikroskopobjekt zur Anwendung kommt, dessen Bildfeld viel zu klein ist, um den in der Hornhaut abzuarbeitenden Bereich vollständig zu erfassen. Das Mikroskopobjekt und damit das Bildfeld wird deshalb bei diesem Laserkeratom verschoben. Eine gekrümmte Schnittfläche kann damit nicht mit vertretbarem Aufwand erzeugt werden, weshalb dieses Laserkeratom die Vorderfläche der Augenhornhaut durch ein planares Kontaktglas flachdrückt, sodass die erzeugte Schnittfläche automatisch parallel zur Augenhornhautvorderfläche liegt. Die Erfindung verwendet hingegen ein ruhendes Bildfeld, für das im Hinblick auf den zu bearbeitenden Bereich ein Durchmesser von mindestens 3 mm, bevorzugt mindestens 6 mm und besonders bevorzugt mindestens 7 mm sinnvoll ist. Kleinere Bildfelder erschweren die Durchführung der Schnittflächenerzeugung bzw. sind für übliche zu isolierende Lentikel zu klein.

Da die Schnittfläche durch Verschieben des Fokus innerhalb des Bildfeldes entlang einer Bahnkurve erfolgt, die in der Schnittfläche liegt, kann bei geeigneter dreidimensionaler Fokuslagenverstellung nahezu jede beliebige gekrümmte Schnittfläche in der Augenhornhaut erzeugt werden. Es ist deshalb nicht mehr erforderlich, die Augenhornhaut flachzudrücken. Vielmehr kann mit einem gekrümmten Kontaktglas gearbeitet werden, wobei es zu bevorzugen ist, dass eine auf die Vorderfläche der Hornhaut aufzusetzende Kontaktfläche dieses Kontaktglases einen Krümmungsradius von nicht über 50 mm, bevorzugt nicht über 20 mm hat. Die Krümmung der Kontaktfläche des Kontaktglases gibt die Krümmung der Hornhaut während des Eingriffs vor.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Insbesondere ist die Erfindung in leicht abgewandelter Form auch für die Erzeugung von gekrümmten Schnitten in anderen Elementen des Auges vorteilhaft anwendbar, beispielsweise in der Linse oder dem Glaskörper.

Nachfolgend wird die Erfindung beispielsweise anhand der beigefügten Zeichnungen, die auch erfindungswesentliche Merkmale offenbaren, noch näher erläutert. Es zeigen:
- Fig. 1: eine Schemadarstellung einer Vorrichtung für die Durchführung einer lentikelextrahierenden Fehlsichtigkeitskorrektur,
- Fig. 2: eine vereinfachte Schnittdarstellung durch die Augenhornhaut zur Veranschaulichung des zu isolierenden und extrahierenden Lentikels und
- Fig. 3: eine Projektion einer das Lentikel begrenzenden Schnittfläche zur Verdeutlichung der Schnittflächenerzeugung.

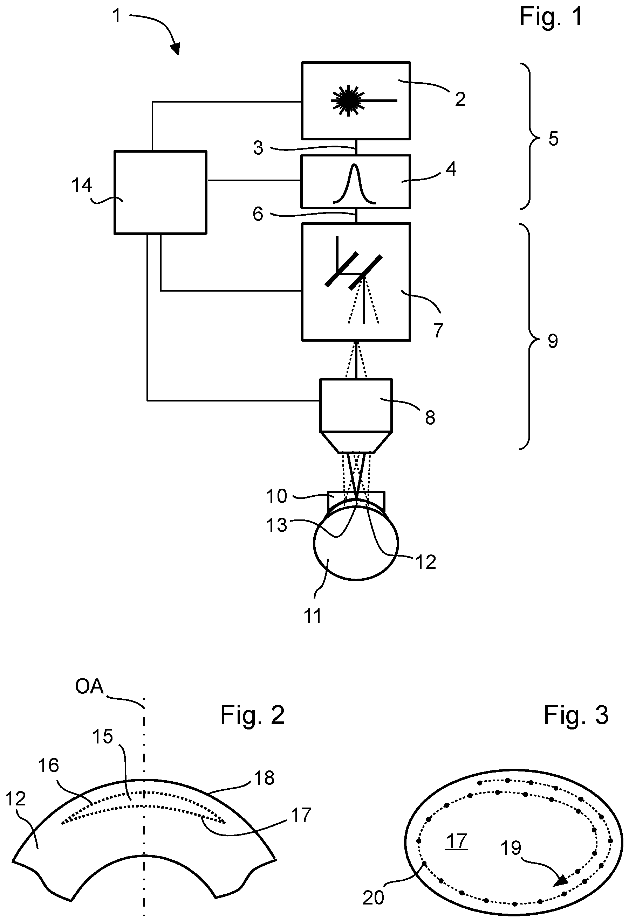

Fig. 1 zeigt schematisch eine Vorrichtung 1 zum Ausführen einer lentikelextrahierenden Fehlsichtigkeitskorrektur. Die Vorrichtung 1 weist einen Laser 2 auf, der gepulste Laserstrahlung bereitstellt, wobei in der geschilderten Ausführungsform der Laser 2 einen gepulsten Rohstrahl 3 abgibt, der eine Wellenlänge hat, welche in das Gewebe der Augenhornhaut eindringt, sodass dort eine Bearbeitung mittels nichtlinearer Effekte erfolgen kann. Der Rohstrahl 3 wird von einem Pulsformer 4 hinsichtlich der Pulsdauer geformt, wobei eine aus dem Stand der Technik bekannte Vorverzerrung erfolgen kann, die dafür sorgt, dass nach Durchlauf durch den weiteren optischen Weg des Strahlengangs im Material, d. h. in der Hornhaut des Auges die gewünschte Pulslänge von beispielsweise ≤ 1 ps vorliegt. Der Pulsformer 4 und der Laser 2 bilden zusammen eine Laserstrahlquelle 5, welche einen gepulsten Laserstrahl 6 der gewünschten Pulslänge abgibt.

Der gepulste Laserstrahl 6 fällt weiter durch einen Scanner 7, welcher bevorzugt eine zweidimensionale Ablenkung quer zur Ausbreitungsrichtung der Laserstrahlung bewirkt. Der derart gescannte Laserstrahl 6 wird von einem Objektiv 8 in die Augenhornhaut fokussiert. Der Scanner 7 bildet zusammen mit dem Objektiv 8 eine Strahlformungseinrichtung 9, die dafür sorgt, dass die gepulste Laserstrahlung 6 an einstellbare Orte in eine Hornhaut eines Auges fokussiert wird, wobei der Fokusdurchmesser dort weniger als 3 µm beträgt.

Die Laserstrahlquelle 5 kann optional einen sogenannten Pulspicker umfassen, der beispielsweise Bestandteil des Pulsformers 4 sein kann. Dieser Pulspicker verändert den Rohstrahl 3 hinsichtlich der Frequenz der Laserstrahlungspulse, die in der Augenhornhaut einen Bearbeitungseffekt haben. So ist es beispielsweise möglich, den Laser 2 so zu auszubilden, dass er einen Rohstrahl 3 mit einer Pulsfrequenz bereitstellt, die deutlich höher ist, als diejenige Pulsfrequenz, die für die bearbeitungswirksamen Laserpulse des Laserstrahls 6 gewünscht ist. Der Pulspicker reduziert dann die Frequenz der wirksamen Laserstrahlungspulse, indem er einzelne Laserstrahlungspulse hinsichtlich ihrer Bearbeitungswirkung unschädlich macht. Dies kann beispielsweise dadurch erfolgen, dass der Pulspicker die Pulslänge vergrößert. Die Bedeutung des Pulspickers, der im Stand der Technik bekannt ist, wird später noch anhand der Fig. 3 erläutert werden.

In der in Fig. 1 gezeigten Ausführungsform beträgt die Pulsfrequenz des gepulsten Laserstrahls 6 zwischen 1,2 MHz und 10 MHz, wobei die Frequenz auf diejenigen Pulse bezogen sind, die einen Bearbeitungseffekt haben, d. h. Pulse, die nicht von einem gegebenenfalls vorhandenen Pulspicker unschädlich gemacht wurden.

Die Energie dieser Pulse des gepulsten Laserstrahls 6 liegt zwischen 10 nJ und 80 nJ.

Die Wellenlänge des Laserstrahls 6 liegt in einem Bereich von 1030 nm bis 1060 nm oder von 300 nm bis 400 nm oder einem anderen Spektralbereich, der in die Hornhaut eindringen kann, für den die Hornhaut also einen Transmissionsgrad von mindestens 0,8 hat.

Die Vorrichtung 1 umfasst weiter ein Kontaktglas 10, das zur Fixierung des Auges 11 dient und auch dazu, um einer Vorderfläche der Hornhaut 12 des Auges 11 eine gewünschte und bekannte Form zu verleihen. Die entsprechende Kontaktfläche des Kontaktglases 10 hat dazu einen Krümmungsradius von 50 mm oder kleiner, besonderes bevorzugt 20 mm oder kleiner.

Das Objektiv 8 bündelt die Laserstrahlung 6 in einen Fokus 13, der innerhalb der Hornhaut 12 liegt. Der Fokus 13 hat einen maximalen Durchmesser von 3 µm, bevorzugt von maximal 2 µm. Der maximale Durchmesser ist dabei der größte Durchmesser, der z. B. im Falle eines elliptischen Fokusflecks entlang der großen Halbachse gemessen wird. Im Falle eines kreisförmigen Spots ist der Spot-Durchmesser das relevante Maß.

Fig. 1 zeigt gestrichelt, dass je nach Wirkung des Scanners 7 der Fokus 13 an verschiedenen Stellen in der Hornhaut 12 des Auges 11 liegt. Der Scanner 7 bewirkt in der Bauweise der Fig. 1 eine Ablenkung quer zur Haupteinfallsrichtung der Laserstrahlung 6. Eine Verstellung der Fokusposition längs der Haupteinfallsrichtung erfolgt durch geeignete Ansteuerung des Objektivs 8, das für eine z-Verstellung geeignet ausgebildet ist.

Die Laserstrahlquelle 5 (in der Bauweise der Fig. 1 realisiert durch Laser 2 und Pulsformer 4) sowie die Strahlformungseinrichtung 9 (in der Bauweise der Fig. 1 realisiert durch Scanner 7 und Objektiv 8) sind über nicht weiter bezeichnete Steuerleitungen mit einem Steuergerät 14 verbunden, das diese Elemente geeignet ansteuert. Das Steuergerät 14 erzeugt durch die Ansteuerung eine Schnittfläche in der Augenhornhaut. Die entsprechenden Verhältnisse sind in einer Schnittdarstellung in Fig. 2 dargestellt, welche schematisch die Hornhaut 12 zeigt.

Durch die Verstellung des Fokus 13 der gepulsten Laserstrahlung 6 wird ein Lentikel 15 in der Hornhaut 12 isoliert. Das Lentikel 15 ist anterior durch eine Flap-Fläche 16 und posterior durch eine Lentikel-Fläche 17 begrenzt. Um die Grenzflächen des Lentikels 15 möglichst einfach halten zu können, befindet sich die Flap-Fläche 16 in konstantem Abstand zur Vorderfläche 18 der Hornhaut 12. Die Flap-Fläche 16 ist somit nicht gegenüber der Vorderfläche 18 gekrümmt. Dies ist bei der Lentikel-Fläche 17 anders, die gegenüber der Vorderfläche 18 gekrümmt ist. Ohne eine solche Krümmung würde die Entnahme des Lentikels 15 die Krümmung der Vorderfläche 18 der Hornhaut 12 nicht ändern. Die gegenüber der Vorderfläche 18 gekrümmte Lentikel-Fläche 17 stellt hingegen eine Änderung der Krümmung der Vorderfläche 18 der Hornhaut 12 ein, wenn das Lentikel 15 entnommen wird. Diese Entnahme geschieht durch einen in der Fig. 2 nicht dargestellten seitlichen Schnitt, der beispielsweise am Rand des Lentikels 15 von der Flap-Fläche 16 zur Vorderfläche 18 führt und es erlaubt, das isolierte Lentikel 15 zu extrahieren, gegebenenfalls nach vorheriger Zerkleinerung des Materials des Lentikels 15. In der Darstellung der Fig. 2 sind Flap-Fläche 16 und Lentikel-Fläche 17 symmetrisch zur optischen Achse OA. Dies stellt sich für die Flap-Fläche 16 automatisch ein, wenn sie konstanten Abstand zur Vorderfläche 18 hat.

Die Grenzflächen des Lentikels 15 können außer der Flap-Fläche 16 und der Lentikel-Fläche 17 natürlich noch weitere Flächen umfassen. Beispielsweise kann bei einem Lentikel 15, das auf der optischen Achse OA dünner ist, als in achsenfernen Bereichen, eine zusätzliche Randfläche vorgesehen sein, welche die Flap-Fläche 16 mit der Lentikel-Fläche 17 verbindet, die dann einen stärker gekrümmten Verlauf aufweist, als die Flap-Fläche 16 und die Vorderfläche 18.

Die Schnittflächen zum Isolieren des Lentikels 15 werden dadurch erzeugt, dass der Fokus 13 entlang einer Bahn verschoben wird, die in der entsprechenden Fläche liegt. Dies ist exemplarisch in Fig. 3 anhand der Lentikel-Fläche 17 gezeigt, die aus Anschaulichkeitsgründen hier elliptisch ist. Dies soll zeigen, dass mit der Vorrichtung 1 nicht nur ein sphärischer Sehfehler, sondern auch ein Astigmatismus korrigiert werden kann. Grundsätzlich ist bei der Korrektur höherer Aberrationen das Lentikel 15 nicht mehr rotationssymmetrisch zur optischen Achse OA. Fig. 3 zeigt eine Auffaltung der Lentikel-Fläche 17 in die Zeichnungsebene. Gestrichelt ist in Fig. 3 eine Bahn 19 eingetragen. Entlang dieser Bahn wird die Position des Fokus 13 verstellt. Dabei ist natürlich in der Regel nicht nur eine Verstellung quer zur optischen Achse OA nötig, sondern auch eine Verstellung der Fokusposition längs der optischen Achse OA. Dies ist in Fig. 3 deshalb nicht zu erkennen, da diese Figur eine Auffaltung der Lentikel-Fläche 17 in die Zeichnungsebene zeigt, weshalb die Bahn 19 in der Darstellung der Fig. 3 in einer Ebene liegt. Betrachtet man sich den Schnitt durch das Lentikel 15 in Fig. 2, wird deutlich, dass mit zunehmendem Abstand von der optischen Achse OA auch die z-Position des Fokus von der Vorderfläche 18 hin weggeschoben wird.

Entlang der Bahn 19 der Fig. 3 sind Zielpunkte 20 eingetragen. Sie bezeichnen jeweils einen Punkt, auf den ein Laserpuls der gepulsten Laserstrahlung 6 abgegeben wird. Durch Aneinanderreihung der Zielpunkte 20 entlang der Bahn 19 und durch geeignete Wahl der Bahn 19 wird insgesamt die Lentikel-Fläche 17 als Schnittfläche ausgebildet. Die Abstände der Zielpunkte 20 sind dabei so gewählt, dass möglichst keine Materialbrücken stehen bleiben, die Lentikel-Fläche 17 also vollflächig als Schnittfläche generiert ist.

Anhand Fig. 3 ist leicht zu verstehen, warum es vorteilhaft ist, die Pulsfrequenz des gepulsten Laserstrahls 6 veränderbar zu machen. Möchte man die Zielpunkte 20 möglichst äquidistant anordnen, sind die Pulsfrequenz und die Verschiebgeschwindigkeit der Stahlformungseinrichtung 9 aufeinander anzupassen. Da ein Laser 2 bei hoher Pulsfrequenz in der Regel nur unter großem Aufwand verstellbar ist, ist es von Vorteil, mit dem Laser 2 zuerst einen Rohstrahl 3 bereitzustellen, der eine Pulsfrequenz hat, welche größer gleich der maximalen Pulsfrequenz ist, die für den Laserstrahl 6 gewünscht wird. Es kann einfacher sein, einen solchen Laser 2 zu realisieren und mit einem Pulspicker zu kombinieren, als einen Laser aufzubauen, der hinsichtlich seiner Pulsfrequenz direkt verstellbar ist. So kann die Pulsfrequenz an die Verschiebegeschwindigkeit angepasst werden und man minimiert die Dauer der Schnittflächenerzeugung.

Die in dieser Figurenbeschreibung sowie im allgemeinen Teil der Beschreibung genannten Parameter für Pulsenergie, Pulsfrequenz, Fokusdurchmesser und optional Pulsdauer führen dazu, dass die Schnittflächen mit einem Mechanismus der Gewebetrennung erzeugt werden, der das Gewebeschneiden und im Wesentlichen die Gewebespaltung ausnützt. Damit kann eine gewünschte Lage für die begrenzenden Schnittflächen des Lentikels 15 hochgenau realisiert werden.

## Patentansprüche

1. Vorrichtung zum Isolieren eines Lentikels (15) in der Hornhaut (12) eines Auges (11), die aufweist
- eine Laserstrahlquelle (5), die ausgebildet ist, gepulste Laserstrahlung (6) abzugeben mit
- einer Pulsfrequenz von 1,2 MHz bis 10 MHz,
- einer Pulsenergie von 1 nJ bis 200 nJ und
- einer in die Hornhaut (12) eindringenden Wellenlänge,
- einer Strahlformungseinrichtung (9), die aufweist
- eine Strahloptik (8), welche die gepulste Laserstrahlung (6) in die Hornhaut (12) in einen Fokus (13) bündelt, der einen maximalen Durchmesser unter 3 µm hat, und
- eine Strahlablenkeinrichtung (7), die den Fokus (13) in der Hornhaut (12) verschiebt, wobei der Fokus (13) entlang einer Bahn (19) wandert,
- eine Steuereinrichtung (14), die ausgebildet ist, die Laserstrahlquelle (5) und die Strahlformungseinrichtung (9) anzusteuern, um in der Hornhaut (12) durch die Vorgabe der Bahn (19) das Lentikel zu isolieren, welches durch mindestens eine Schnittfläche (17) begrenzt ist,
die gegenüber einer Vorderfläche (18) der Hornhaut (12) gekrümmt ist,
**dadurch gekennzeichnet, dass**
- die Laserstrahlquelle (5) ausgebildet ist, die gepulste Laserstrahlung (6) mit einer Pulsenergie von 10 nJ bis 80 nJ abzugeben und/oder die Strahloptik ein Objektiv (8) mit numerischer Apertur von mindestens 0,33 aufweist.

2. Vorrichtung nach Anspruch 1, wobei die Laserstrahlquelle (5) ausgebildet ist, die gepulste Laserstrahlung (6) mit einer Wellenlänge von 1030 nm bis 1060 nm abzugeben.

3. Vorrichtung nach Anspruch 2, wobei die Laserstrahlquelle (5) ausgebildet ist, die gepulste Laserstrahlung (6) mit einer Pluslänge unter 1 ps abzugeben.

4. Vorrichtung nach eine Anspruch 1, wobei die Laserstrahlquelle (5) ausgebildet ist, die gepulste Laserstrahlung (6) mit einer Wellenlänge von 300 nm bis 400 nm und einer Pulsfrequenz nicht über 2 MHz abzugeben, und die Strahloptik die gepulste Laserstrahlung in den Fokus (13) bündelt, der einen maximalen Durchmesser kleiner oder gleich 2 µm hat.

5. Vorrichtung nach einem der obigen Ansprüche, die ein Kontaktglas (10) zum Aufsetzen auf die Hornhaut (12) aufweist, wobei eine auf die Vorderfläche (18) der Hornhaut (12) aufzusetzende Kontaktfläche des Kontaktglases (10) einen Krümmungsradius nicht über 50 mm, bevorzugt nicht über 20 mm hat.

6. Vorrichtung nach einem der obigen Ansprüche, wobei die Strahloptik (8) in der Hornhaut (12) des Auges (11) ein optisches Feld mit einem Durchmesser von ≥ 3 mm, insbesondere von ≥ 6 mm und besonders bevorzugt von ≥ 7 mm hat.

7. Verfahren zum Isolieren eines Lentikels (15) in der Hornhaut (12) eines Auges (11), wobei
- in der Hornhaut (12) mindestens eine Schnittfläche (16, 17) festgelegt wird, die das Lentikel (15) umgrenzt und die nicht in einem konstanten Abstand zur Vorderfläche (18) der Hornhaut (12) verläuft,
- eine Bahn (19) festgelegt wird, die in der Schnittfläche ( 17) liegt,
- in die Hornhaut (12) gepulste Laserstrahlung (6) abgegeben wird mit
- einer Pulsfrequenz von 1,2 MHz bis 10 MHz,
- einer Pulsenergie von 1 nJ bis 200 nJ und
- einer in die Hornhaut (12) eindringenden Wellenlänge,
- eine Strahloptik (8) verwendet wird, welche die gepulste Laserstrahlung (6) in die Hornhaut (12) in einen Fokus (13) bündelt, der einen maximalen Durchmesser unter 3 µm hat, und
- der Fokus (13) in der Hornhaut (12) verschoben wird, wobei der Fokus (13) entlang der Bahn (19) wandert
- wobei das Verfahren keine chirurgische Behandlung des lebenden menschlichen oder tierischen Auges ausführt,
**dadurch gekennzeichnet, dass**
- die Pulsenergie einen Wert von 10 nJ bis 80 nJ hat und/oder die Strahloptik (8) eine numerische Apertur von mindestens 0,33 hat.

8. Verfahren nach Anspruch 7, wobei die Wellenlänge einen Wert von 1030 nm bis 1060 nm hat.

9. Verfahren nach Anspruch 8, wobei die Pluslänge unter 1 ps liegt.

10. Verfahren nach einem der Ansprüche 7 bis 8, wobei die Wellenlänge einen Wert von 300 nm bis 400 nm, die Pulsfrequenz einen Wert nicht über 2 MHz und der Fokus einen maximalen Durchmesser kleiner oder gleich 2 µm hat.

11. Verfahren nach einem der Ansprüche 7 bis 10, wobei ein Kontaktglas (10) auf das Auge aufgesetzt wird, das eine auf der Vorderfläche (18) der Hornhaut (12) aufgesetzte Kontaktfläche mit einem Krümmungsradius nicht über 50 mm, bevorzugt nicht über 20 mm hat.

12. Verfahren nach einem der Ansprüche 7 bis 11, wobei die Strahloptik (8) in der Hornhaut (12) des Auges (11) ein optisches Feld mit einem Durchmesser von ≥ 3 mm, insbesondere von ≥ 6 mm und besonders bevorzugt von ≥ 7 mm hat.

## Claims

1. An apparatus for isolating a lenticule (15) in the cornea (12) of an eye (11), comprising
- a laser beam source (5) configured to emit pulsed laser radiation (6) with
- a pulse frequency of 1.2 MHz to 10 MHz,
- a pulse energy of 1 nJ to 200 nJ, and
- a wavelength that penetrates the cornea (12),
- a beamforming device (9) comprising
- beam optics (8) that focus the pulsed laser radiation (6) into the cornea (12) at a focus (13) having a maximum diameter of less than 3 µm, and
- a beam deflection device (7) that shifts the focus (13) within the cornea (12), wherein the focus (13) travels along a path (19),
- a control device (14) configured to control the laser beam source (5) and the beamforming device (9) to isolate the lenticule in the cornea (12) by specifying the path (19), which lenticule is bounded by at least one cut surface (17) that is curved relative to a front surface (18) of the cornea (12),
**characterized in that**
- the laser beam source (5) is configured to emit the pulsed laser radiation (6) with a pulse energy of 10 nJ to 80 nJ and/or the beam optics comprise an objective (8) with a numerical aperture of at least 0.33.

2. The apparatus according to claim 1, wherein the laser beam source (5) is configured to emit the pulsed laser radiation (6) with a wavelength of 1030 nm to 1060 nm.

3. The apparatus according to claim 2, wherein the laser beam source (5) is configured to emit the pulsed laser radiation (6) with a pulse width of less than 1 ps.

4. The apparatus according to claim 1, wherein the laser beam source (5) is configured to emit the pulsed laser radiation (6) with a wavelength of 300 nm to 400 nm and a pulse frequency not exceeding 2 MHz, and the optical system focuses the pulsed laser radiation into a focal spot (13) having a maximum diameter of 2 µm or less.

5. The apparatus according to one of the preceding claims, comprising a contact glass (10) for placement on the cornea (12), wherein a contact surface of the contact glass (10) to be placed on the front surface (18) of the cornea (12) has a radius of curvature not exceeding 50 mm, preferably not exceeding 20 mm.

6. The apparatus according to any of the preceding claims, wherein the beam optics (8) have an optical field in the cornea (12) of the eye (11) with a diameter of ≥ 3 mm, in particular of ≥ 6 mm, and most preferably of ≥ 7 mm.

7. A method for isolating a lenticule (15) in the cornea (12) of an eye (11), wherein
- at least one cut surface (17) is defined in the cornea (12) that delimits the lenticule (15) and does not extend at a constant distance from the anterior surface (18) of the cornea (12),
- a path (19) is defined that lies along the cut surface (17),
- pulsed laser radiation (6) is emitted into the cornea (12) with
- a pulse frequency of 1.2 MHz to 10 MHz,
- a pulse energy of 1 nJ to 200 nJ, and
- a wavelength penetrating into the cornea (12),
- beam optics (8) are used, which focus the pulsed laser radiation (6) into the cornea (12) at a focus (13) having a maximum diameter of less than 3 µm, and
- the focus (13) is shifted within the cornea (12), wherein the focus (13) travels along the path (19),
- wherein the method does not perform surgical treatment of the living human or animal eye, **characterized in that**
- the pulse energy has a value of 10 nJ to 80 nJ and/or the beam optics (8) have a numerical aperture of at least 0.33.

8. The method according to claim 7, wherein the wavelength has a value of 1030 nm to 1060 nm.

9. The method according to claim 8, wherein the pulse duration is less than 1 ps.

10. The method according to any one of claims 7 to 8, wherein the wavelength has a value of 300 nm to 400 nm, the pulse frequency has a value not exceeding 2 MHz, and the focus has a maximum diameter of 2 µm or less.

11. The method according to any one of claims 7 to 10, wherein a contact glass (10) is placed on the eye, which has a contact surface placed on the front surface (18) of the cornea (12) with a radius of curvature not exceeding 50 mm, preferably not exceeding 20 mm.

12. The method according to any one of claims 7 to 11, wherein the beam optics (8) produce an optical field in the cornea (12) of the eye (11) with a diameter of ≥ 3 mm, in particular of ≥ 6 mm, and most preferably of ≥ 7 mm.

## Revendications

1. Appareil à isoler une lentille (15) dans la cornée (12) d'un œil (11), comprenant
- une source de faisceau laser (5) configurée pour émettre un rayonnement laser pulsé (6) ayant
- une fréquence d'impulsion comprise entre 1,2 MHz et 10 MHz,
- une énergie d'impulsion comprise entre 1 nJ et 200 nJ, et
- une longueur d'onde qui pénètre dans la cornée (12),
- un dispositif de formation de faisceau (9) comprenant
- une optique de faisceau (8) qui focalise le rayonnement laser pulsé (6) dans la cornée (12) en un foyer (13) ayant un diamètre maximal inférieur à 3 µm, et
- un dispositif de déviation de faisceau (7) qui déplace le foyer (13) à l'intérieur de la cornée (12), le foyer (13) se déplaçant le long d'un trajet (19),
- un dispositif de commande (14) configuré pour commander la source de faisceau laser (5) et le dispositif de formation de faisceau (9) afin d'isoler le lenticule dans la cornée (12) en spécifiant le trajet (19), lequel lenticule est délimité par au moins une surface de coupe (17) qui est courbée par rapport à une surface avant (18) de la cornée (12),
**caractérisé en ce que**
- la source de faisceau laser (5) est configurée pour émettre le rayonnement laser pulsé (6) avec une énergie d'impulsion de 10 nJ à 80 nJ et/ou l'optique de faisceau comprend un objectif (8) avec une ouverture numérique d'au moins 0,33.

2. Appareil selon la revendication 1, dans lequel la source de faisceau laser (5) est configurée pour émettre le rayonnement laser pulsé (6) avec une longueur d'onde comprise entre 1 030 nm et 1 060 nm.

3. Appareil selon la revendication 2, dans lequel la source de faisceau laser (5) est configurée pour émettre le rayonnement laser pulsé (6) avec une largeur d'impulsion inférieure à 1 ps.

4. Appareil I selon la revendication 1, dans lequel la source de faisceau laser (5) est configurée pour émettre un rayonnement laser pulsé (6) ayant une longueur d'onde comprise entre 300 nm et 400 nm et une fréquence d'impulsion ne dépassant pas 2 MHz, et le système optique focalise le rayonnement laser pulsé en un point focal (13) ayant un diamètre maximal de 2 µm ou moins.

5. Appareil selon l'une des revendications précédentes, comprenant une lentille de contact (10) destinée à être placée sur la cornée (12), dans lequel une surface de contact de la lentille de contact (10) destinée à être placée sur la surface antérieure (18) de la cornée (12) présente un rayon de courbure ne dépassant pas 50 mm, de préférence ne dépassant pas 20 mm.

6. Appareil selon l'une quelconque des revendications précédentes, dans lequel l'optique de faisceau (8) présente un champ optique dans la cornée (12) de l'œil (11) d'un diamètre ≥ 3 mm, en particulier ≥ 6 mm, et de préférence ≥ 7 mm.

7. Procédé pour isoler un lenticule (15) dans la cornée (12) d'un œil (11), dans lequel
- au moins une surface de coupe (17) est définie dans la cornée (12) qui délimite le lenticule (15) et ne s'étend pas à une distance constante de la surface antérieure (18) de la cornée (12),
- un trajet (19) est défini qui s'étend le long de la surface de coupe (17),
- un rayonnement laser pulsé (6) est émis dans la cornée (12) avec
- une fréquence d'impulsion de 1,2 MHz à 10 MHz,
- une énergie d'impulsion de 1 nJ à 200 nJ, et
- une longueur d'onde pénétrant dans la cornée (12),
- on utilise une optique de faisceau (8) qui focalise le rayonnement laser pulsé (6) dans la cornée (12) en un foyer (13) ayant un diamètre maximal inférieur à 3 µm, et
- le foyer (13) est déplacé à l'intérieur de la cornée (12), le foyer (13) se déplaçant le long du trajet (19),
- dans lequel le procédé n'effectue pas de traitement chirurgical de l'œil vivant humain ou animal,
**caractérisé en ce que**
- l'énergie d'impulsion a une valeur comprise entre 10 nJ et 80 nJ et/ou l'optique de faisceau (8) a une ouverture numérique d'au moins 0,33.

8. Procédé selon la revendication 7, dans lequel la longueur d'onde a une valeur comprise entre 1030 nm et 1060 nm.

9. Procédé selon la revendication 8, dans lequel la durée d'impulsion est inférieure à 1 ps.

10. Procédé selon l'une quelconque des revendications 7 à 8, dans lequel la longueur d'onde a une valeur comprise entre 300 nm et 400 nm, la fréquence d'impulsion a une valeur ne dépassant pas 2 MHz, et le foyer a un diamètre maximal de 2 µm ou moins.

11. Procédé selon l'une quelconque des revendications 7 à 10, dans lequel un verre de contact (10) est placé sur l'œil, dont la surface de contact est placée sur la face antérieure (18) de la cornée (12) avec un rayon de courbure ne dépassant pas 50 mm, de préférence ne dépassant pas 20 mm.

12. Procédé selon l'une quelconque des revendications 7 à 11, dans lequel l'optique de faisceau (8) produit un champ optique dans la cornée (12) de l'œil (11) d'un diamètre ≥ 3 mm, en particulier ≥ 6 mm, et de préférence ≥ 7 mm.
